(19) Europäisches Patentamt
European Patent Office
Office européen des brevets

(11) Veröffentlichungsnummer: **0 050 869**
**B1**

(12) # EUROPÄISCHE PATENTSCHRIFT

(45) Veröffentlichungstag der Patentschrift: **20.06.84**

(51) Int. Cl.$^3$: **C 07 C 85/20,** C 07 C 87/02, C 07 C 69/06, C 07 C 67/22

(21) Anmeldenummer: **81108898.8**

(22) Anmeldetag: **25.10.81**

(54) **Verfahren zur Herstellung von N-tert.-Alkylaminen oder Cycloalkylaminen und Estern der Ameisensäure.**

(30) Priorität: **27.10.80 DE 3040403**

(43) Veröffentlichungstag der Anmeldung: **05.05.82 Patentblatt 82/18**

(45) Bekanntmachung des Hinweises auf die Patenterteilung: **20.06.84 Patentblatt 84/25**

(84) Benannte Vertragsstaaten: **AT BE CH DE FR GB LI NL**

(56) Entgegenhaltungen:
**DE - B - 1 197 091**
**DE - B - 1 493 433**

(73) Patentinhaber: **Degussa Aktiengesellschaft, Weissfrauenstrasse 9, D-6000 Frankfurt am Main 1 (DE)**

(72) Erfinder: **Lehmann, Bernd, Dr. Dipl.-Chem., Flurstrasse 5, D-6463 Freigericht 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von Aminen und Estern aus Äthern unter Umsetzung der Äther mit Cyanwasserstoff in Gegenwart starker Säuren.

Es sind mehrere Verfahren zur Herstellung von N-tert.-Alkylaminen bekannt. In allen Fällen werden zunächst in einer ersten Verfahrensstufe N-tert.-Alkylformamide gewonnen. Aus diesen werden dann in einer zweiten Verfahrensstufe durch Hydrolyse die N-tert.-Alkylamine erzeugt, wobei gleichzeitig Ameisensäure entsteht.

Bekannt ist, dass bei der Umsetzung von Alkenen mit Cyanwasserstoff in Gegenwart von stark sauren Kondensationsmitteln, wie Schwefelsäure, Formamide entstehen und diese durch Hydrolyse mittels starker Säuren oder Basen in die entsprechenden Amine und Ameisensäure übergeführt werden. Beispielsweise wird aus Isobuten das N-tert.-Butylformamid und aus diesem neben Ameisensäure das N-tert.-Butylamin gebildet. Im Falle der Hydrolyse des Formamids mittels Chlorwasserstoffsäure wird aus dem Umsetzungsgemisch zunächst die Ameisensäure und dann nach Zugabe von Basen das Amin abdestilliert (DE-PS Nr. 870856). Nachteilig ist bei diesem Verfahren, dass die Auseuten mässig sind und überdies die Ameisensäure in Form unreiner verdünnter wässeriger Lösungen anfällt.

Es ist auch bekannt, N-tert.-Alkylformamide durch Umsetzung tertiärer Alkohole oder verzweigter Alkene mit Cyanwasserstoff unter Verwendung von Ameisensäure als Kondensationsmittel zu erzeugen und die Formamide durch Hydrolyse mittels Alkali in die N-tert.-Alkylamine zu überführen (DE-AS Nr. 2236040). Bei diesem Verfahren sind die Umsetzungszeiten für die Bildung der Formamide sehr lang und die erzielten Raum-Zeit-Ausbeuten gering. Brauchbare Ausbeuten werden nur erreicht, wenn der Cyanwasserstoff und insbesondere die Ameisensäure in mehrfach molarem Überschuss eingesetzt werden. Diese im Überschuss angewendeten Substanzen können nur unter erheblichem Aufwand und überdies nur unvollständig zurückgewonnen werden.

Bekannt ist ausserdem, dass aus tert.-Alkyläthern die entsprechenden N-tert.-Alkylformamide entstehen, wenn die Äther mit Cyanwasserstoff in der 4fachen bis 50fachen Menge konzentrierter Säure umgesetzt werden und das Umsetzungsgemisch mit Wasser verdünnt wird (US-PS Nr. 2518156). Dieses Verfahren ist wegen der erforderlichen grossen Menge konzentrierter Säure sehr aufwendig.

Schliesslich ist auch bekannt, das durch Umsetzung von Alkenen mit Cyanwasserstoff in Gegenwart von Schwefelsäure erzeugte tert.-Butylformamid mittels Natriumhydroxid zu hydrolisieren, zunächst aus dem Umsetzungsgemisch das tert.-Butylamin abzudestillieren, dann in dem restlichen Umsetzungsgemisch die Ameisensäure mittels Schwefelsäure freizusetzen und mit Methanol zu verestern und schliesslich den Ameisensäuremethylester abzudestillieren (US-PS Nr. 4131642). Bei diesem Verfahren ist die Ausbeute sowohl an dem tert.-Butylamin als auch an dem Ameisensäureester unbefriedigend.

Es ist nun ein Verfahren zur Herstellung von Aminen und Estern aus Äthern unter Umsetzung der Äther mit Cyanwasserstoff in Gegenwart starker Säuren gefunden worden, das dadurch gekennzeichnet ist, dass tert.-Alkyläther oder Cycloalkyläther primärer oder sekundärer Alkanole in Gegenwart der bis zu etwa zweifachen molaren Menge der starken Säuren zu N-tert.-Alkylaminen oder Cycloalkylaminen und Estern der Ameisensäuren mit primären oder sekundären Alkanolen umgesetzt werden. Bei diesem Verfahren werden unmittelbar aus den Äthern die Amine und gleichzeitig die Ester der Ameisensäure gebildet. Sowohl die Amine als auch die Ester fallen mit ausgezeichneter Ausbeute an.

Das erfindungsgemässe Verfahren ist besonders geeignet für die Umsetzung von Äthern der allgemeinen Formel

$$\begin{array}{c} R_1 \\ | \\ R_2-C-O-R_4 \\ | \\ R_3 \end{array} \qquad (I)$$

in der $R_1$, $R_2$ und $R_3$ gleich oder verschieden sind und unverzweigte oder verzweigte, gegebenenfalls substituierte, Alkylgruppen mit 1 bis 10, vorzugsweise 1 bis 4, Kohlenstoffatomen, insbesondere Methylgruppen, bedeuten oder $R_1$ und $R_2$ gemeinsam mit dem C-Atom, an das sie gebunden sind, ein – gegebenenfalls substituierter – Cycloalkylring mit 5 bis 12, vorzugsweise 5 bis 8, Kohlenstoffatomen im Ring, insbesondere ein Cyclohexanring, sind und $R_3$ Wasserstoff oder eine unverzweigte oder verzweigte – gegebenenfalls substituierte und gegebenenfalls mit $R_1$ oder $R_2$ zu einem Ring geschlossene – Alkylgruppe mit 1 bis 10, vorzugsweise 1 bis 4, Kohlenstoffatomen, insbesondere eine Methylgruppen, ist, und $R_4$ eine unverzweigte oder verzweigte Alkylgruppe mit 1 bis 10 Kohlenstoffatomen, vorzugsweise eine Propylgruppe, Isopropylgruppe oder Äthylgruppe, insbesondere eine Methylgruppe, ist, mit Cyanwasserstoff zu Aminen der allgemeinen Formel

$$\begin{array}{c} R_1 \\ | \\ R_2-C-NH_2 \\ | \\ R_3 \end{array} \qquad (II)$$

in der $R_1$, $R_2$ und $R_3$ die zuvor angegebenen Bedeutungen haben, und Ameisensäureestern der allgemeinen Formel

$$\begin{array}{l} HC-O-R_4 \\ \;\;\| \\ \;\;O \end{array} \qquad (III)$$

in der $R_4$ die zuvor angegebene Bedeutung hat.

Als Äther kommen beispielsweise tert.-Butylpropyläther, tert.-Butylisopropyläther, tert.-Pentyläthyläther, tert.-Pentylisopropyläther, tert.-Pentylpropyläther, (1,1-Dimethylbutyl)methyläther, (1,1-Dimethylbutyl)äthyläther, (1-Äthyl-1-methylbutyl)methyläther, (1-Äthyl-1,3-dimethylbutyl)methyläther, (1,1,3,3-Tetramethylbutyl)-methyläther, (1,1,3,3-Tetramethylbutyl)äthyläther, Methoxycyclopentan, Äthoxycyclopentan, Methoxycyclohexan, Äthoxycyclohexan, Methoxycycloheptan, Äthoxycycloheptan, Methoxycyclooctan, 1-Methoxy-1-methylcyclohexan, 1-Methoxy-1-methylcyclopentan, Bornylmethyläther, Methylnorbonyläther, Menthylmethyläther, Adamanthylmethyläther, insbesondere tert.-Butyläthyläther, tert.-Butylisopropyläther, tert.-Butylpropyläther und tert.-Amylmethyläther in Frage. Mit besonderem Vorteil ist das erfindungsgemässe Verfahren für die Umsetzung des tert.-Butylmethyläthers geeignet.

Statt Cyanwasserstoff können Substanzen verwendet werden, die unter den Umsetzungsbedingungen Cyanwasserstoff liefern, beispielsweise Salze der Cyanwasserstoffsäure, wie Natriumcyanid, oder Cyanhydrine, wie Acetoncyanhydrin.

Als starke Säuren werden beispielsweise Monoester der Schwefelsäure, zweckmässigerweise mit einem Alkanol, das der allgemeinen Formel

$$HO-R_4 \quad (IV)$$

entspricht, in der $R_4$ die zuvor genannten Bedeutungen hat, oder Sulfonsäuren, wie Methansulfonsäure oder Benzolsulfonsäure, eingesetzt. Besonders geeignet ist Schwefelsäure.

In welchen Mengenverhältnissen die Substanzen angewendet werden und welche Umsetzungsbedingungen, wie Temperatur und Druck, gewählt werden, richtet sich gegebenenfalls nach der Art der Substanzen.

Je Mol Äther werden bis zu etwa 2 mol der starken Säuren eingesetzt. Im allgemeinen ist es zweckmässig, etwa 0,8 bis 1,8 mol der Säuren zu nehmen. Vorzugsweise werden 1,0 bis 1,3 – insbesondere 1,05 bis 1,15 – mol der Säuren je Mol Äther angewendet.

Das Mengenverhältnis Äther zu Cyanwasserstoff kann weitgehend beliebig gewählt werden, jedoch ist es in den meisten Fällen vorteilhaft, nicht weniger als etwa 0,8 und nicht mehr als etwa 2,0 mol Cyanwassersotoff je Mol Äther zu nehmen. Vorzugsweise werden 1,0 bis 1,2 mol Cyanwasserstoff je Mol Äther angewendet. Anstelle von Cyanwasserstoff können diesem äquivalente Mengen der Cyanwasserstoff abgebenden Substanzen eingesetzt werden. In diesem Fall wird zusätzlich eine entsprechende Menge starker Säure benötigt.

Zur Durchführung des erfindungsgemässen Verfahrens ist es erforderlich, Wasser zuzusetzen. Dieses kann als solches oder mit den übrigen Substanzen eingebracht werden. Wenngleich die Wassermenge weitgehend beliebig gewählt werden kann, ist es im allgemeinen zweckmässig, insgesamt nicht mehr als etwa 4 mol Wasser je Mol Äther zu nehmen, zu Beginn der Umsetzung jedoch nicht mehr als etwa 1 mol. Vorzugsweise werden etwa 0,9 bis 2,0 mol Wasser je Mol Äther angewendet.

Gegebenenfalls kann es zweckmässig sein, je Mol Äther bis zu etwa 1 mol, in manchen Fällen bis zu etwa 3 mol, eines Alkohols zuzufügen. Besonders vorteilhaft ist, ein Alkanol zu verwenden, das der allgemeinen Formel IV entspricht.

Zweckmässig ist es im allgemeinen, bei Temperaturen etwa zwischen −30 und +100°C zu arbeiten. Vorteilhaft sind in den meisten Fällen Temperaturen zwischen 20 und 80°C. Es kann zweckmässig sein, die Umsetzung bei Temperaturen bis zu etwa 50°C zu beginnen und bei höheren Temperaturen zu beenden, wobei mit Vorteil zunächst Temperaturen gewählt werden, die unterhalb des Siedepunktes des Umsetzungsgemisches liegen, und im weiteren Verlauf das Umsetzungsgemisch zum Sieden gebracht wird.

Wenngleich der Druck weitgehend beliebig gewählt werden kann, ist es vorteilhaft, vom Normaldruck nicht wesentlich abzuweichen. In manchen Fällen kann es wegen des Vorliegens flüchtiger Substanzen zweckmässig sein, bei einem der Temperatur entsprechenden höheren Druck zu arbeiten.

Eine bevorzugte Verfahrensweise ist, die Ausgangssubstanzen, den Äther, den Cyanwasserstoff und die starke Säure, in wasserfreier oder weitgehend wasserfreier Form einzusetzen, die Umsetzung zunächst bei niedrigen Temperaturen, die unterhalb des Siedepunktes des Umsetzungsgemischs liegen, durchzuführen, dann das Wasser und gegebenenfalls den Alkohol zuzusetzen und schliesslich das Umsetzungsgemisch zum Sieden zu bringen.

Aus dem Umsetzungsgemisch kann das Amin und der Ester in an sich bekannter Weise abgetrennt und gewonnen werden, wobei sich die Verfahrensweise gegebenenfalls nach der Art der Substanzen richtet. Vorteilhaft ist es in vielen Fällen, beispielsweise bei der Herstellung des tert.-Butylamins oder tert.-Amylamins einerseits und der Ester der Ameisensäure mit Propanol (1), Propanol-(2), Äthanol oder Methanol andererseits, zunächst den Ester aus dem Umsetzungsgemisch abzutreiben, dann im restlichen Umsetzungsgemisch mittels alkalisch wirkender Substanzen das Amin freizusetzen und schliesslich dieses abzutreiben.

*Beispiel 1*

Eine Lösung von 54,1 g (2,0 mol) Cyanwasserstoff in 176,4 g (2,0 mol) tert.-Butylmethyläther wurde im Verlauf von 30 min in 225 g 96%ige Schwefelsäure (2,2 mol) eingetragen. Die Mischung wurde 15 min lang auf 50°C gehalten, mit 45 g Wasser und 45 g Methanol versetzt und weitere 60 min lang unter Rückfluss auf Siedetemperatur gehalten. Aus dem Umsetzungsgemisch wurde zunächst Ameisensäuremethylester abgetrieben. Es wurden 113 g des Esters gewonnen, entsprechend 94% Ausbeute, bezogen auf den eingesetzten Äther. Dem verbliebenen Umsetzungsgemisch wurden 100 g Wasser zugesetzt. Dann wurden 45 g Methanol abgetrieben. Schliesslich wurde nach Zugabe von 391 g einer 45%igen wässerigen Natriumhydroxydlösung tert.-Butylamin abdestilliert. Es wurden 133 g des

Amins gewonnen, entsprechend 91% Ausbeute, bezogen auf den eingesetzten Äther. Der Siedepunkt des Amins war 44°C.

*Beispiel 2*

Es wurde wie nach Beispiel 1 verfahren, doch wurde nicht die Schwefelsäure, sondern die Lösung aus Cyanwasserstoff in dem tert.-Butylmethyläther vorgelegt und in diese die Schwefelsäure eingetragen. Die Ausbeute betrug 112 g Ameisensäuremethylester, entsprechend 93%, und 134 g tert.-Butylamin, entsprechend 92%, bezogen auf den eingesetzten Äther.

*Beispiel 3*

166,4 g 96%ige Schwefelsäure (1,6 mol) wurden in eine Lösung von 40,6 g (1,5 mol) Cyanwasserstoff in 132,3 g (1,5 mol) tert.-Butylmethyläther eingetragen. Die Mischung wurde hierbei von 20 auf 50°C erwärmt, dann 15 min auf 50°C gehalten, mit 35 ml Wasser versetzt und 90 min lang unter Rückfluss auf Siedetemperatur gehalten. Bei der Abtreibung des Ameisensäuremethylesters wurden gegen Ende weitere 50 ml Wasser zugesetzt. Die Abtreibung des tert.-Butylamins erfolgte nach Zugabe von 290 g 45%iger wässeriger Natriumhydroxydlösung. Die Ausbeute betrug 76 g Ameisensäuremethylester, entsprechend 84%, und 94 g tert.-Butylamin, entsprechend 86%, bezogen auf den eingesetzten Äther.

*Beispiel 4*

Eine Lösung von 85,1 g (1,0 mol) Acetoncyanhydrin in 88,2 g (1,0 mol) tert.-Butylmethyläther wurde in 98 g (1,0 mol) wasserfreie Schwefelsäure getropft. Die Temperatur wurde hierbei und weitere 15 min lang auf 25°C gehalten. Dann wurden 37 g Aceton abgetrieben und 18 g Wasser und 20 g Methanol zugesetzt. Danach wurde der Ameisensäuremethylester abgetrieben, der Rückstand mit 187 g 45%iger wässeriger Natriumhydroxydlösung versetzt und schliesslich das tert.-Butylamin abgetrieben. Die Ausbeute betrug 49 g Ameisensäuremethylester, entsprechend 82%, und 40 g tert.-Butylamin, entsprechend 55%, bezogen auf den eingesetzten Äther.

*Beispiel 5*

Eine Lösung von 59,4 g (2,2 mol) Cyanwasserstoff in 176,4 g (2,0 mol) tert.-Butylmethyläther wurde in 232,2 g einer 84,5%igen Schwefelsäure (2,0 mol Schwefelsäure und 2,0 mol Wasser) eingetragen. Die Temperatur wurde währenddessen und weiter 60 min lang auf 25°C gehalten. Der Mischung wurden dann 43 g Methanol zugesetzt, und es wurde im übrigen wie nach Beispiel 1 verfahren. Die Ausbeute betrug 113,5 g Ameisensäuremethylester, entsprechend 95%, und 118 g tert.-Butylamin, entsprechend 81%, bezogen auf den eingesetzten Äther.

*Beispiel 6*

204,4 g 96%ige Schwefelsäure (2,0 mol) wurden in eine Lösung von 54,1 g (2,0 mol) Cyanwasserstoff in 204,4 g (2,0 mol) tert.-Amylmethyläther eingetragen. Die Mischung wurde währenddessen auf 25°C und danach 60 min lang auf 50°C gehalten, dann mit 45 g Wasser und 60 g Methanol versetzt und 60 min lang unter Rückfluss auf Siedetemperatur gehalten. Aus dem Umsetzungsgemisch wurde zunächst der Ameisensäuremethylester, nach Zugabe von 200 ml Wasser das überschüssige Methanol und schliesslich nach Zugabe von 178 g 45%iger Natriumhydroxydlösung das tert.-Amylamin abgetrieben. Der Siedepunkt des Amylamins war 77°C. Die Ausbeute betrug 114 g Ameisensäuremethylester, entsprechend 95%, und 167 g tert.-Amylamin (Reinheit 95%), entsprechend 91%, bezogen auf den eingesetzten Äther.

*Beispiel 7*

Es wurde wie nach Beispiel 6 verfahren, jedoch statt der Natriumhydroxydlösung eine Suspension von 148 g Calciumhydroxyd in 780 ml Wasser zugesetzt. Die Ausbeute an tert.-Amylamin (Reinheit 93%) betrug 168 g, entsprechend 90%.

*Beispiel 8*

Es wurde wie nach Beispiel 6 verfahren, jedoch wurde tert.-Butyläthyläther eingesetzt. Statt Methanol wurde eine entsprechende Menge Äthanol verwendet. Die Ausbeute an Ameisensäureäthylester betrug 89%, die an tert.-Butylamin 88%, bezogen auf den eingesetzten Äther.

*Beispiel 9*

Es wurde wie nach Beispiel 6 verfahren, jedoch wurde tert.-Butylisopropyläther eingesetzt. Statt Methanol wurde eine entsprechende Menge Propanol-(2) verwendet. Die Ausbeute an Ameisensäureisopropylester betrug 94%, die an tert.-Butylamin 90%, bezogen auf den eingesetzten Äther.

*Beispiel 10*

Es wurde wie nach Beispiel 6 verfahren, jedoch wurde tert.-Butyl-n-propyläther eingesetzt. Statt Methanol wurde eine entsprechende Menge Propanol-(1) verwendet. Die Ausbeute an Ameisensäurepropylester betrug 82%, die an tert.-Butylamin 84%, bezogen auf den eingesetzten Äther.

*Beispeil 11*

153,3 g 96%ige Schwefelsäure (1,5 mol) wurden in eine Lösung von 27,0 g (1,0 mol) Cyanwasserstoff in 128,0 g (1,0 mol) 1-Methoxy-1-methylcyclohexan eingetragen. Die Mischung wurde währenddessen auf 25°C und danach 60 min lang auf 50°C gehalten, dann mit 22 g Wasser und 30 g Methanol versetzt und 60 min lang unter Rückfluss auf Siedetemperatur gehalten. Aus dem Umsetzungsgemisch wurde zunächst der Ameisensäuremethylester und nach Zugabe von 100 ml Wasser das überschüssige Methanol abgetrieben. Schliesslich wurden 267 g einer 45%igen Natriumhydroxydlösung zugesetzt. Es bildeten sich zwei Phasen, von denen die obere 1-Methylcyclohexylamin war. Die untere Phase wurde mit Diäthyläther extrahiert, und der Extrakt wurde mit der oberen Phase vereinigt. Diese wurde dann mit Natriumsulfat getrocknet und zur Gewinnung des Amins destilliert. Der Siedepunkt des Amins war 142°C. Die Ausbeute betrug 57 g Ameisensäuremethylester, entsprechend 95%, und 91 g 1-Methylcyclohexylamin (Reinheit 96%), entsprechend 77%, bezogen auf den eingesetzten Äther.

## Patentansprüche

1. Verfahren zur Herstellung von N-tert.-Alkylaminen oder Cycloalkylaminen und Estern der Ameisensäure aus Äthern unter Umsetzung der Äther mit Cyanwasserstoff und Wasser in Gegenwart starker Säuren, dadurch gekennzeichnet, dass tert.-Alkyläther oder Cycloalkyläther primärer oder sekundärer Alkanole in Gegenwart der bis zu etwa zweifachen molaren Menge der starken Säuren zu N-tert.-Alkylaminen oder Cycloalkylaminen und Estern der Ameisensäure mit primären oder sekundären Alkanolen umgesetzt werden.

2. Verfahren nach Anspruch 1, dadurch gekennzeichnet, dass 1,05 bis 1,15 mol Säure je Mol Äther angewendet wird.

3. Verfahren nach einem der beiden Ansprüche 1 oder 2, dadurch gekennzeichnet, dass als Säure Schwefelsäure verwendet wird.

4. Verfahren nach den Ansprüchen 1 bis 3, dadurch gekennzeichnet, dass aus dem Umsetzungsgemisch zuerst der Ameisensäureester und dann das Amin abgetrennt wird.

## Claims

1. A process for the production of N-tertiary-alkylamines or cycloalkylamines and esters of formic acid from ethers by reacting the ethers with hydrogen cyanide and water in the presence of strong acids, characterised in that tertiary-alkylethers or cycloalkylethers of primary or secondary alkanols are reacted in the presence of up to about double the molar quantity of the strong acids to produce N-tertiary-alkylamines or cycloalkylamines and formic acid esters of the primary or secondary alkanols.

2. A process according to claim 1, characterised in that from 1.05 to 1.15 mol of acid are used per mol of ether.

3. A process according to claim 1 or 2, characterised in that sulphuric acid is used as acid.

4. A process according to claims 1 to 3, characterised in that first of all the formic acid ester is separated and then the amine is separated from the reaction mixture.

## Revendications

1. Procédé pour la préparation de N-tert.-alcoylamines ou de cycloalcoylamines et d'esters de l'acide formique, à partir d'éthers, par réaction de l'éther avec de l'acide cyanhydrique et de l'eau en présence d'acides forts, caractérisé en ce que les tert.-alcoyléther ou cycloalcoyléther d'alcanols primaires ou secondaires sont amenés à réagir, en présence de quantités qui peuvent aller jusqu'au double de la proportion molaire d'acides forts pour former des N-tert.-alcoylamines ou cycloalcoylamines et des esters de l'acide formique avec des alcanols primaires ou secondaires.

2. Procédé suivant la revendication 1, caractérisé en ce que l'on utilise 1,05 à 1,15 mol d'acide par mole d'éther.

3. Procédé suivant l'une des revendications 1 ou 2, caractérisé en ce que, comme acide, on utilise l'acide sulfurique.

4. Procédé suivant l'une des revendications 1 à 3, caractérisé en ce que l'on sépare d'abord, du mélange réactionnel, l'ester de l'acide formique et, ensuite, l'amine.